Europäisches Patentamt

European Patent Office

Office européen des brevets

(11) Publication number: **0 305 346 B1**

# EUROPEAN PATENT SPECIFICATION

(45) Date of publication of patent specification: **30.12.92** (51) Int. Cl.⁵: **A61L  33/00**

(21) Application number: **88850268.9**

(22) Date of filing: **18.08.88**

Consolidated with 88907440.7/0379503
(European application No./publication No.) by
decision dated 30.12.91.

(54) **Articles exhibiting a blood-compatible surface layer and process for providing articles with such a surface layer.**

(30) Priority: **26.08.87 SE 8703310**

(43) Date of publication of application:
**01.03.89 Bulletin  89/09**

(45) Publication of the grant of the patent:
**30.12.92 Bulletin  92/53**

(84) Designated Contracting States:
**AT BE CH DE ES FR GB GR IT LI LU NL SE**

(56) References cited:
CA-A- 1 208 557        JP-A-60 202 702
JP-A-61 206 452        JP-A-62 053 666
US-A- 4 279 795        US-A- 4 530 974
US-A- 4 553 973        US-A- 4 708 951

(73) Proprietor: **Astra Tech Aktiebolag
Box 14
S-431 21 Mölndal(SE)**

(72) Inventor: **Nygren, Bo Hakan
Valebergsvägen 4C
S-427 00 Billdal(SE)**
Inventor: **Stenberg, Johan Emanuel
Hedelundsvägen 5
S-417 43 Göteborg(SE)**

(74) Representative: **Hjertman, Ivan T. et al
AB ASTRA Patent and Trade Mark Depart-
ment
S-151 85 Södertälje(SE)**

EP 0 305 346 B1

## Description

Field of the invention

The present invention relates to articles exhibiting a blood-compatible surface layer and methods for providing such articles with such a surface layer and in particular for providing articles useful within medicine. More particularly, the invention relates to articles exhibiting at least one surface of glass, metal or a hydrophobic polymer coated with a blood-compatible surface layer and processes for providing articles exhibiting at least one surface of glass, metal or a hydrophobic polymer with a coating of a blood-compatible surface layer, as well as the use of substrate material of glass, metal or hydrophobic polymer, coated with the surface layer of an adsorbed ethyldroxyethyl-cellulose which has been made hydrophobic and has a flocculation temperature of 35-40°C for manufacture of a medical article having a blood-compatible surface.

Background of the invention

Prior art technique to provide articles useful in medicine with a blood-compatible surface layer often comprises an alteration in the surface energy of the material. An improvement in the properties of various materials has been obtained by modifying the surface layers either to a more hydrophobic character or to a more hydrophilic character. Rendering the surface layer hydrophobic, for instance by the methylization of a glass surface, results in a decrease in the effectiveness of the surface activated coagulation system of the blood. However, proteins such as fibrinogen are bound comparatively firmly to such surface and to this protein layer certain cells, the thrombocytes, can be bound and activated whereafter coagulation is started even though it proceeds slowly. Hydrophilic surfaces, e.g. hydrolysed nylon or oxidized aluminium, have presented reduced binding of cells but the surface activated coagulation system is not prevented at these surfaces. The use of these surfaces in contact with blood thus implies the addition of anti-coagulants, for instance heparin to the blood.

Another prior art surface treatment technique for preventing coagulation comprises binding of anticoagulants into the surface layer. Heparin has primarily been used with this technique. Heparin is a hexoseaminehexuronic acid polysaccharide which is sulphatized and has acid properties, i.e. heparin is an organic acid. According to DE-A-21 54 542 articles of an organic thermoplastic resin is first impregnated with an amino-silane coupling agent and the article thus treated is then reacted with an acid solution of heparin salt to the binding of heparin in the surface layer by means of ionic bonds. Surfaces thus treated with heparin have proved to reduce the coagulation reaction. A considerable disadvantage of these surfaces, however, is that the heparin treatment does not prevent the adherence of thrombocytes, which is a great problem in, for instance, heart-lung machines.

On the 10th Annual Meeting of the Society for Biomaterials (Washington D.C. April 27, 1984) was described that polyethyleneglycol surfaces on quartz minimize protein adsorption. Procedures for covalent binding of polyethylenglycol to surfaces have previously been described, e.g. in WO-A-86/02087. Polyion complexes formed between a cationic and an anionic cellulose derivative have also been found to have good blood-compatibilities (Ito, H. et.al., J. Appl. Polym. Sci., Vol. 32 (1986) 3413). Methods of covalent binding of water-soluble polymers to surfaces have also been described, e.g. in EP-A-166 998.

It is known that water-binding gels, for instance polyhydroxyalkylmethacrylate, reduce the adsorption of proteins and present a low adhesiveness to cells (Hoffman et al., Ann. N.Y. Acad. Sci., Vol. 283 (1977) 372). These properties are considered to be due to the fact that gels containing water give a low surface energy in the interface to the blood. The prior art technique for manufacturing of water-binding gels, however, is impaired by disadvantages such as complicated preparation technique and incomplete polymerization, which results in leakage of toxic monomers. A gel-like mixture of sacccharose and glucose included in a matrix of the polysaccharide dextran or dextrin is used in accordance with previously known technique as a tube for the connection of blood-vessels. This mixture should have the effect that no toxicity to the patient occurs that the implantate is dissolved in the blood after some time. It is known that the neutral polysaccharide dextran is miscible with blood without provoking any coagulation reaction. Dextran has been used as a surface coating on glass, aluminium and silicon rubber, and has been shown to reduce blood coagulation during blood contact with these surfaces as described in WO83/03977.

The adhesion of blood components to surfaces in contact with blood could be decreased by preadsorption of albumin to hydrophobic surfaces (Mosher, D.F in: Interaction of blood with natural and artificial surfaces, Ed. Salzman, E.W, Dekker Inc 1981). The adsorbed albumin does not form a stable coating, but is desorbed during contact with blood and coagulation is induced although at a lower rate.

The Canadian patent CA-A-1 208 557 relates to an article, viz a tag for attachment to a wound. Said tag can be made of polycarbonate or polyurethane for instance. The tag is coated with a water- and serumsoluble polymeric material e.g. hydroxy(lower)alkyl cellulose such as hydroxypropylcellulose containing an antiseptic. Said coating slowly dissolves when coming into contact with blood, liberating the antiseptic to prevent infections.

Abstract No. 67 368 of Chemical Abstracts, Vol. 102 (1985) discloses a study of the correlation between the surface properties and the antithrombogenicity of polymeric materials, among which cellulose derivatives are mentioned. Said abstract shows that different polymers in contact with blood induces different thrombocyte aggregation.

Derwent Abstract No. 87-239975/34 refers to a macromolecular material per se. Said material comprises aminoethyl cellulose as a main chain and poly-gamma-benzyl-L-glutamate or poly-N5-2-hydroxyalkyl-L-glutamine as a side chain.

Derwent Abstract No. 87-105705/15 relates to an anti-coagulating material which consists of a mixture of a hydrophobic polymer and a hydrophilic polymer, wherein the hydrophilic polymer is made by ionic binding a sulphonated polyallylamine to the quaternary amino group of an acrylic polymer containing a quarternary amino nitrogen, and which is suitable for use in catheters, artificial internal organs such as valves, blood vessel, kidneys, and lungs, and for blood storage vessels, injectors, etc.

Derwent Abstract No. 87-282129/43 relates to a material obtained by mixing a polyurethane or polyurethane-urea having a hydrophilic polyalkyleneether part. Said material is used for the manufacturing of blood vessel catheters, bypass tubes, artificial lungs, etc.

Derwent Abstract No. 87-293554/47 describes a dialysis membrane comprising a crosslinked PVA type hydrophilic high molecular compound and a fluoro-methacrylate hydrophobic polymer. The article described therein is a membrane.

In Journal of Bioengineering, Vol.2, pp 241-249, 1978, two procedures have been examined for altering the blood compatibility of copolymers of dimethylaminoethyl methacrylate, where blood compatibility has been measured by the recalcification time of platelet rich plasma. The improvement in blood compatibility of a copolymer of dimethylaminomethyl methacrylate and methyl methacrylate resulting from contact with heparin has been shown to be dependent on both copolymer composition and the method of polymerization.

US patent US-A-4 553 973 discloses an osmotic delivery system for dispensing a useful agent. The system comprises a semipermeabel wall surrounding a compartment housing the agent. The wall comprises a cellulose ether, an organic solvent soluble polymer and optionally an aqueous soluble polymer.

US patent US-A-4 708 951 (published November 24, 1987) discloses an anticoagulative high-molecular composition which comprises a cellulosic polyelectrolyte complex formed from a polycationic cellulose derivative such as a quaternary ammonium salt of hydroxyalkyl cellulose, and a polyanionic cellulose such as sodium cellulose glycolate, which is used in the field of medical instruments or devices.

Disclosure of the invention

The object of the present invention is to provide articles useful within medicine with a blood-compatible surface layer. This means, for articles intended for use in contact with blood, that the article which is alien to the blood is treated in such a way that it does not induce coagulation or formation of thromboses.

The present invention affords technique for the surface treatment of material important for medical technology, such as glass, metal and hydrophobic polymers (e.g. polytetrafluoroethylene (PTFE)).

The article according to the invention which exhibits at least one surface of glass, metal or hydrophobic polymer coated with a blood-compatible surface layer, is characterized in that the blood-compatible surface layer consists of an adsorbed ethylhydroxyethylcellulose which has been made hydrophobic and has a flocculation temperature of 35-40°C.

The process according to the invention for providing articles exhibiting at least one surface of glass, metal or hydrophobic polymer coated with blood-compatible surface layer, is characterized in that said substrate surface of the article, after hydrophobization when required, is exposed to a solution of ethylhydroxyethylcellulose which has been made hydrophobic and has a flocculation temperature of 35-40°C, and which adsorbs to said substrate surface.

The substrate surface has to be hydrophobic before coating. For metals or metal oxides this can be achieved by methylization with silanes.

The ethylhydroxyethylcellulose which has been made hydrophobic used in the present invention is limited soluble in water depending on the degree of hydrophobization.

The ethylhydroxyethylcellulose used in this invention is characterized by the property of the ability to adsorb at hydrophobic surfaces. The hydrophobization of said polymer can be performed by binding of hydrocarbons to the polymer backbone. Examples of such hydrocarbons are alkyl-groups, benzyl-groups or alkenyl-groups. The hydrophobization renders the above polymer partly insoluble in water with a flocculation above a certain temperature or above a certain ionic strength.

The substrate surface is exposed to a solution of the ethylhydroxyethylcellulose at a temperature below flocculation and at a salt concentration below flocculation. The ethylhydroxyethylcellulose which has been made hydrophobic, adsorbs strongly to hydrophobic surfaces. The above ethylhydroxyethylcellulose has a flocculation temperature of 35-40°C.

The treated surface proves biologically inert and surfaces treated in this way give reduced adsorption of proteins, adherence of cells and coagulation. The adsorbed polymer is not exchanged by plasma proteins.

The process according to the present invention can be applied within many fields. Thus, in heart-lung machines there are used many details which are made of aluminum which can easily be treated by methyl-silane to get a hydrophobic surface.

The process according to the present invention is ideally suited for treatment of venous catheters. These are often manufactured in PTFE and this material is normally not blood compatible. For this material it is also difficult to find appropriate processes for covalent coupling of hydrophilic polymers.

The invention may also be applied in other connections, for instance, for treatment of articles of hydrophobic plastics for sampling and/or storage of blood.

The invention will in the following be illustrated by a working example but is not limited thereto and hence modifications are of course conceivable within the limits of the claims.

### Working example

### Coating procedure

a) The polymer is purified and isolated by repeated heat flocculation and centrifugation.

b) A polytetraflouroethylene-tube (teflon), diameter 3 mm, and a polyurethane-tube, diameter 3 mm, were immersed in a solution of ethyl-hydroxyethyl-cellulose (EHEC, 1 g/l, prepared according to US-A-3,926,951) in distilled water for 20 hours in room temperature. The tubes were rinsed in saline for 1 minute.

### Experimental tests

Two different tests were performed. Incubation with a solution of fibrinogen at high concentration was performed in order to detect exchange reactions between the polymer and plasma protein. Incubation with whole blood followed by measurement of released thromboglobulin was used to measure the stability of the polymer coating and the activation of platelets at the surface.

### Fibrinogen adsorption

a) Coated and non-coated tubes were incubated in a solution of human fibrinogen (1 g/l) in saline for 30 minutes at room temperature.

b) The tubes were rinsed in saline for 10 seconds.

c) Incubation in anti-fibrinogen antiserum diluted 1:1000 for 1 hour at room temperature.

d) Incubation with peroxidase-conjugated anti-antibodies for 30 minutes.

e) Incubation in a solution of orthophenylenediamine (0.5g/l) and 0.01% $H_2O_2$ in 0.1 M citrate buffer, pH = 4.5.

f) Addition of 2 M $H_2SO_4$ and reading of absorbance at $\lambda = 450$ nm.

### Blood-compatibility test

a) Venous blood (18 ml) was tapped from a healthy donor into 2.0 ml of a solution of hirudin in saline (500 IE/ml) (Hirudin is a thrombin inactivator).

b) The blood was filled into coated and non-coated tubes and allowed to incubate for 2 hours at room temperature.

c) 1 ml of blood was drawn into a syringe containing 0.2 ml diatube® and the mixture was centrifugated at 5000 g for 30 minutes at +3°C.

d) The supernatant was collected and the amount of $\beta$-thromboglobuline using a commercial kit

EP 0 305 346 B1

(Diagnostica Stago®).

Results

The results of the tests above are presented in table I and II. Table I shows that the amount of fibrinogen adsorbed to the tubes is reduced by the coating with ethyl-hydroxyethyl-cellulose. Table II shows that the amount of $\beta$-thromboglobuline released from the platelets during incubation with blood is also reduced by the coating with ethyl-hydroxyethyl-cellulose.

TABLE I

| Amount of surface adsorbed fibrinogen as measured by the ELISA | | | | | | | |
|---|---|---|---|---|---|---|---|
| | teflon | polyurethane | polypropene | latex rubber | polyvinyl | silicone rubber | polystyrene |
| uncoated | 0.953 | 0.868 | 0.406 | 0.220 | 0.439 | 0.794 | 0.634 |
| EHEC (35°C)* | 0.242 | 0.188 | 0.304 | 0.110 | 0.268 | 0.246 | 0.005 |
| EHEC (45°C)* | 0.969 | 0.420 | 0.308 | 0.153 | 0.274 | 0.560 | 0.016 |
| Klucel®** (<RT)* | 1.042 | 0.922 | 0.434 | 0.131 | 0.203 | 0.722 | |

Footnotes:
*flocculation temperature of the polymer in distilled water.
**propyl-hydroxypropylcellulose

TABLE II

| Amount of $\beta$-thromboglobuline released as measured by the ELISA | | |
|---|---|---|
| | teflon | polyurethane |
| uncoated | 1.38 | 1.67 |
| EHEC (35°C) | 0.44 | 0.85 |

**Claims**

1. Article exhibiting at least one hydrophobic surface of glass, metal or a hydrophobic polymer coated with a blood compatible surface layer, characterized in that the blood compatible surface layer consists of an adsorbed ethylhydroxyethylcellulose, which has been made hydrophobic and a has a flocculation temperature of 35-40°C.

2. Article according to claim 1, characterized in that the hydrophobic polymer surface is polytetrafluoroethylene.

3. Article according to claims 1-2, characterized in that it is a catheter, tube or a device for sampling and/or storage of blood.

4. Article according to claims 1-2, characterized in that it is a heart-lung machine exhibiting at least one hydrophobic aluminium surface with a coating of a blood compatible surface layer.

5. Process for providing articles exhibiting at least one hydrophobic surface of glass, metal or a hydrophobic polymer with a coating of a blood compatible surface layer, characterized in that said surface of the article, after hydrophobization when required, is exposed to a solution of ethylhydroxyethylcellulose, which has been made hydrophobic and has a flocculation temperature of 35-40°C, at a temperature below the flocculation.

6. Process according to claim 5 wherein the hydrophobic surface is polytetrafluoroethylene.

5

7. Use of a substrate material of glass, metal or hydrophobic polymer coated with a surface layer consisting of an adsorbed ethylhydroxyethylcellulose, which has been made hydrophobic and has a flocculation temperature of 35-40°C for manufacture of a medical article having a blood compatible surface.

8. Use according to claim 7 wherein the substrate is polytetrafluoroethylene.

**Patentansprüche**

1. Artikel mit mindestens einer hydrophoben Oberfläche aus Glas, Metall oder einem hydrophoben Polymer, überzogen mit einer blutkompatiblen Oberflächenschicht, dadurch gekennzeichnet, daß die blutkompatible Oberflächenschicht aus einer adsorbierten Ethylhydroxyethylcellulose besteht, die hydrophob gemacht wurde und eine Flockungstemperatur von 35-40°C aufweist.

2. Artikel nach Anspruch 1, dadurch gekennzeichnet, daß die hydrophobe Polymeroberfläche Polytetrafluorethylen ist.

3. Artikel nach den Ansprüchen 1 bis 2, dadurch gekennzeichnet, daß er ein Katheter, ein Röhrchen oder eine Einrichtung zum Abnehmen und/oder Aufbewahren von Blut ist.

4. Artikel nach den Ansprüchen 1 bis 2, dadurch gekennzeichnet, daß er eine Herz-Lungen-Maschine mit mindestens einer hydrophoben Aluminiumoberfläche mit einem Überzug aus einer blutkompatiblen Oberflächenschicht ist.

5. Verfahren zur Herstellung von Artikeln mit mindestens einer hydrophoben Oberfläche aus Glas, Metall oder einem hydrophoben Polymer, überzogen mit einer blutkompatiblen Oberflächenschicht, dadurch gekennzeichnet, daß die Oberfläche des Artikels, gegebenenfalls nach einer Hydrophobisierung, mit einer Lösung aus Ethylhydroxyethylcellulose, die hydrophob gemacht wurde und eine Flockungstemperatur von 35 bis 40°C aufweist, bei einer Temperatur unterhalb der Flockungstemperatur behandelt wird.

6. Verfahren nach Anspruch 5, worin die hydrophobe Oberfläche Polytetrafluorethylen ist.

7. Verwendung eines Substratmaterials aus Glas, Metall oder hydrophobem Polymer, überzogen mit einer Oberflächenschicht, bestehend aus einer adsorbierten Ethylhydroxyethylcellulose, die hydrophob gemacht wurde und eine Flockungstemperatur von 35 bis 40°C aufweist, zur Herstellung eines medizinischen Artikels mit einer blutkompatiblen Oberfläche.

8. Verwendung nach Anspruch 7, worin das Substrat Polytetrafluorethylen ist.

**Revendications**

1. Article présentant au moins une surface hydrophobe de verre, de métal ou d'un polymère hydrophobe revêtue d'une couche superficielle compatible avec le sang, caractérisé en ce que la couche superficielle compatible avec le sang est constituée d'une éthylhydroxyéthylcellulose adsorbée qui a été rendue hydrophobe et présente une température de floculation de 35-40°C.

2. Article selon la revendication 1, caractérisé en ce que la surface du polymère hydrophobe est constituée de polytétrafluoréthylène.

3. Article selon les revendications 1-2, caractérisé en ce qu'il s'agit d'un cathéter, d'un tube ou d'un dispositif de prélèvement et/ou de stockage de sang.

4. Article selon les revendications 1-2, caractérisé en ce qu'il s'agit d'un appareil de stimulation coeur-poumons présentant au moins une surface d'aluminium hydrophobe revêtue d'une couche superficielle compatible avec le sang.

5. Procédé pour fournir des articles présentant au moins une surface hydrophobe de verre, de métal ou d'un polymère hydrophobe d'un revêtement superficiel compatible avec le sang, caractérisé en ce que ladite surface de l'article, après traitement hydrophobe lorsque cela s'avère nécessaire, est exposée à une solution d'éthylhydroxyéthylcellulose, qui a été rendue hydrophobe et présente une température de floculation de 35-40°C, à une température inférieure à la température de floculation.

6. Procédé selon la revendication 5, caractérisé en ce que la surface hydrophobe est constituée de polytétrafluoréthylène.

7. Utilisation d'une matière de support de verre, de métal ou d'un polymère hydrophobe revêtue d'une couche superficielle constituée d'une éthylhydroxyéthyl cellulose adsorbée, qui a été rendue hydrophobe et présente une température de floculation de 35-40°C, pour la fabrication d'un article médical ayant une surface compatible avec le sang.

8. Utilisation selon la revendication 7, dans lequel le support est constitué de polytétrafluoréthylène.